# EUROPEAN PATENT SPECIFICATION

(11) **EP 4 008 254 B1**
(45) Date of publication and mention of the grant of the patent: **04.10.2023**
(21) Application number: 20212255.2
(22) Date of filing: 07.12.2020
(51) Int. Cl.: A61B 5/361, A61B 5/00

(54) **METHOD FOR CLASSIFYING ATRIAL FIBRILLATION**
VERFAHREN ZUR KLASSIFIZIERUNG VON VORHOFFLIMMERN
PROCÉDÉ DE CLASSIFICATION DE LA FIBRILLATION AURICULAIRE

(43) Date of publication of application: 08.06.2022
(73) Proprietor: CathVision ApS, 2200 Copenhagen (DK)
(72) Inventor: Paamand, Rune, 3060 Humlebaek (DK); Matthiesen, Mads Emil, 24771 Genarp (SE)
(74) Representative: Gottschald Patentanwälte Partnerschaft mbB

(56) References cited:
- EP-A1- 3 020 333
- WO-A2-2008/035070
- CN-A- 111 449 647
- US-A1- 2013 197 380

## Description

The invention relates to a method for classifying atrial fibrillation by analyzing ECG data via a control system according to the general part of claim 1 and a control system for performing such a method according to claim 16.

Known methods and systems for classifying atrial fibrillation can be found e.g. in US 2013/197380 A1, CN 111 449 647 A, WO 2008/035070 A2, and EP 3 020 333 A1.

Electrically, atrial fibrillation is the chaotic activation of muscle cells of the atria.

During atrial fibrillation, the atria only minimally contribute to the function of the heart. Atrial fibrillation, therefore, reduces the output of the heart but is not imminently dangerous. However, when becoming chronical, atrial fibrillation is correlated with increased morbidity and mortality. One treatment option for atrial fibrillation is ablation therapy. Ablation therapy is the destruction of the cells that allow electrical wave reentry to reduce chaotic activation of the atrial muscle cells.

The success rate of and strategy for atrial fibrillation ablation heavily depends on the level of complexity of the electrical conduction through the atria. If complexity is low, ablation of the usual reentry paths has a high chance of success. If complexity is higher, additional electrical mapping of activation paths can be used. Independent of the treatment, classifying the complexity of atrial fibrillation allows patient-specific treatment and outcome prediction of ablation therapy.

One of the promising methods for classifying atrial fibrillation is calculating the dominant frequency of the electrical activation of the atria from a body surface ecg. Generally said, dominant frequency determination often uses the steps of isolating atrial activation from ECG data and analyzing a frequency spectrum of the remaining atrial signal data. Known methods (US 5,772,604 A) detect a peak frequency in the spectrum of the atrial signal data. While these methods yield promising results, there is still room for improving the classification of atrial fibrillation by providing new classification methods.

It is therefore an object of the present invention to provide an improved method for classifying atrial fibrillation yielding more robust results.

The above-noted problem is solved for a method with the features of the general part of claim 1 by the features of the characterizing part of claim 1.

The main realization of the present invention is that atrial activation is not adequately described by a single peak or main frequency but in itself a complex, instable and rapidly changing chaotic phenomenon. Looking for a single dominant frequency is therefore not an optimal way of describing the physiological reality. The central realization in the present case is that atrial fibrillation can be characterized by searching for a window of frequencies representing the dominant part of the atrial fibrillation.

After decomposing the ECG data thereby generating frequency data comprising a frequency dimension and an amplitude dimension, a dominant window can be found by searching in the frequency dimension for a window meeting an amplitude criterion based on a sum of amplitudes inside the dominant window.

In this way, it becomes possible for a control system to directly search for a part of the frequency dimension, the dominant window, which in particular contains a pre-defined part of the signal power, instead of looking for a single frequency with the maximum power. This method is more robust against random and/or temporary peaks of power in less relevant frequencies.

In detail, it is proposed that in an analysis step the control system identifies in the frequency data a dominant window having a width in the frequency dimension and meeting an amplitude criterion, that the amplitude criterion is based on a sum of amplitudes inside the dominant window, that the control system identifies the dominant window by searching on at least a section, in particular a pre-defined section, of the frequency dimension for a window fulfilling the amplitude criterion and that based on the dominant window the control system determines the classification of atrial fibrillation in the ECG data.

While it is known from previous methods to look for a window around the found dominant frequency, presently the window is found by searching on at least a section of the frequency dimension and not by defining the window around a dominant frequency.

In a preferred embodiment according to claim 2 ventricular components are removed from the ECG data to focus the analysis on fibrillation waves. In another embodiment according to claim 3, the amplitude dimension is an energy density or power density dimension. The frequency distribution of the power of the atrial fibrillation signal inside the ECG is directly related to the complexity of the atrial fibrillation. The same is true for the mathematically dependent signal energy.

Claim 4 names preferred frequency decompositions. All of these methods for frequency decomposition have different strengths and can be used in varying embodiments of the proposed method. The Fourier transformation and the Welch method impose less restrictions on the signal to be analyzed compared to other methods and are therefore also well suited for analyzing the chaotic atrial fibrillation waves. Model-based decompositions like the autoregressive moving average based decomposition again provide the possibility to adapt the decomposition to expected atrial fibrillation waves.

As atrial fibrillation has components that vary rapidly over time, in another preferred embodiment according to claim 5, using a time-frequency decomposition is proposed. In this way, it becomes possible to separate time-stable and non-stable components of the atrial fibrillation waves. In particular, the wavelet transformation allows specifically choosing a wavelet according to expected fibrillation waves and adapting the coarseness of the transformation specifically to the purpose of searching for a dominant window in a section of the frequency dimension.

According to claim 6 in one embodiment, the amplitude criterion may comprise a primary criterion that a sum of amplitudes in the dominant window is at least equal to a percentage of a sum of amplitudes along a part of the frequency dimension. Preferably in this way, the dominant window can be defined as a window containing a pre-defined percentage of the total power of the signal. However, other mathematically related concepts are equally preferred. For example, the power density spectrum could be a normalized or otherwise adapted power density spectrum. In this way, instead of searching for a single power spike, a search for a power band is conducted.

Claim 7 relates to defining the width of the dominant window by using a boundary condition. In this way, further information about the signal can be included in the dominant window. For example, a small dominant window containing a large amount of power may be indicative for a less complex atrial fibrillation.

It may be useful or necessary to identify a single dominant frequency for classifying atrial fibrillation. According to an embodiment described in claim 8, a dominant frequency can be identified based on the dominant window.

Depending on the algorithm used for defining the width of the dominant window, it may be advantageous to analyze the width of the dominant window and classify the atrial fibrillation based on this analysis as described in claim 9. Mostly a wider dominant window implies higher complexity of the atrial fibrillation. Further analysis of the dominant window, as also described in claim 9, may include looking at harmonics of the frequency values of the dominant window as harmonic dominant windows. In particular, the power contained in these harmonic dominant windows may be related to atrial fibrillation complexity.

Further preferred ways of analyzing the frequency data inside the dominant window may include analyzing skewness and/or kurtosis as named in claim 10. Skewness and kurtosis contain additional information about the primary frequencies of atrial fibrillation and their amplitude.

According to an embodiment, described in claim 11, the classification of atrial fibrillation may comprise a presence or absence and preferably a severity indicator of the atrial fibrillation. In particular, the severity of atrial fibrillation is an important factor for deciding for or against ablation therapy and the used ablation method.

Claim 12 relates to the electrical measurement environment with the preferred possibility of using a body-surface ECG. Body-surface ECG data combines high precision with low invasion.

Particularly useful pre-processing steps for the proposed method are named in claim 13. These include removing noise with non-linear filtering and removing powerline interference data. The, preferably subsequent, ventricular component removal step may comprise a QRS or QRST removal as described in claim 14. Either may be combined with a morphology grouping to adapt to varying QRST complex morphology. These variations in QRST complex morphology can for example be caused by the atrial fibrillation itself. As atrial fibrillation inhibits the physiological electrical conduction from the sinus node to the ventricles, ventricular activity during atrial fibrillation may have different physiological causes resulting in two or more sharply different morphology groups each related to their physiological cause.

Further information on the atrial fibrillation may be inferred from additional analysis of the time domain as described in claim 15.

Another teaching according to claim 16, which is of equal importance, is directed to a control system for performing the proposed method. All explanations given with respect to the proposed method are fully applicable.

In the following, an embodiment of the invention is explained with respect to the drawings. The drawings show
- Fig. 1: steps of the proposed method applied in the time domain and
- Fig. 2: a frequency spectrum of the ECG data.

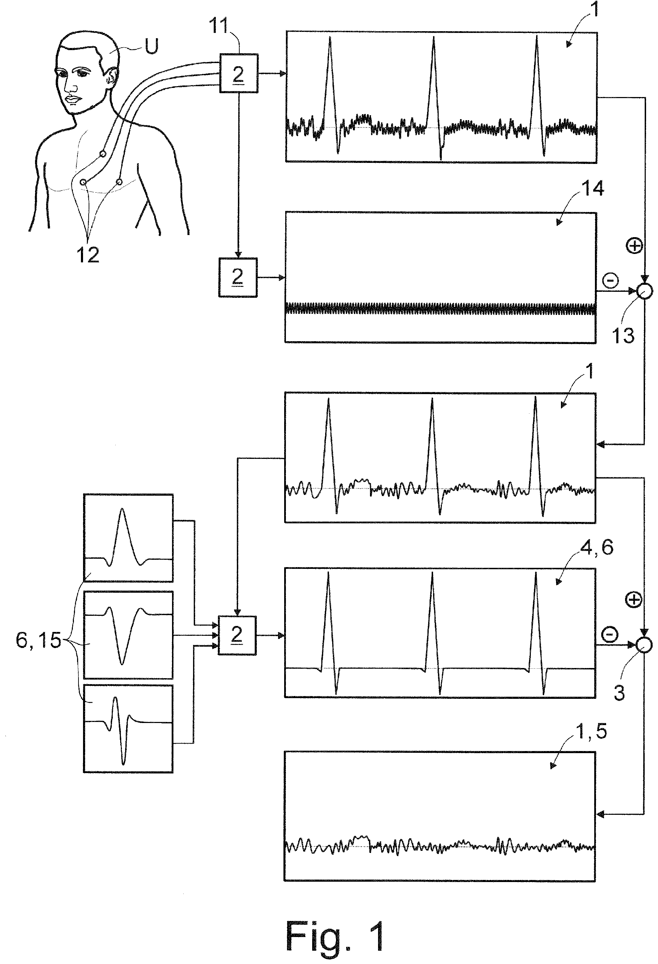

The proposed method is used for classifying atrial fibrillation by analysing ECG data 1 via a control system 2. In Fig. 1 the control system 2 is shown schematically as a control system 2 comprising hardware connected to a user U. In this case, the control system 2 measures the ECG data 1 as a single-channel ECG. However, the ECG data 1 may also comprise one or more channels of a multi-channel ECG. Physically measuring the ECG data 1 is not necessarily comprised by the proposed method, such that the control system 2 may also process ECG data measured by another system separate from the control system 2. The control system 2 therefore also may be a general-purpose computer, cloud-based or the like, and configured to perform the proposed method.

In a preferred embodiment as shown in the figures, the method may comprise a ventricular component removal step 3 in which the control system 2 processes the ECG data 1 to remove ventricular components 4 from the ECG data 1. The ventricular components 4 may be mostly QRS or QRST complex data. As the more powerful part of the activation of the heart, removing the ventricular components 4 from the ECG data 1 allows focussing the analysis onto atrial signal data 5 contained in the ECG data 1. Fig. 1 shows the ventricular component removal step 3 in the bottom part. The top part of Fig. 1 refers to a pre-processing of the ECG data 1 which is explained further below. In this case, the ventricular component removal step 3 comprises a subtraction of a template 6. This will also be explained further below. It is equally preferred to use different methods of removing some or all ventricular components 4. In particular, the ventricular component removal step may comprise the complete removal of sections of the ECG data 1 containing the QRS or QRST complex. The method may then comprise analysing one or more sections of the ECG data between QRS or QRST complexes. To acquire higher frequency resolution, known methods like zero padding or known methods for connecting the signal sections may be used.

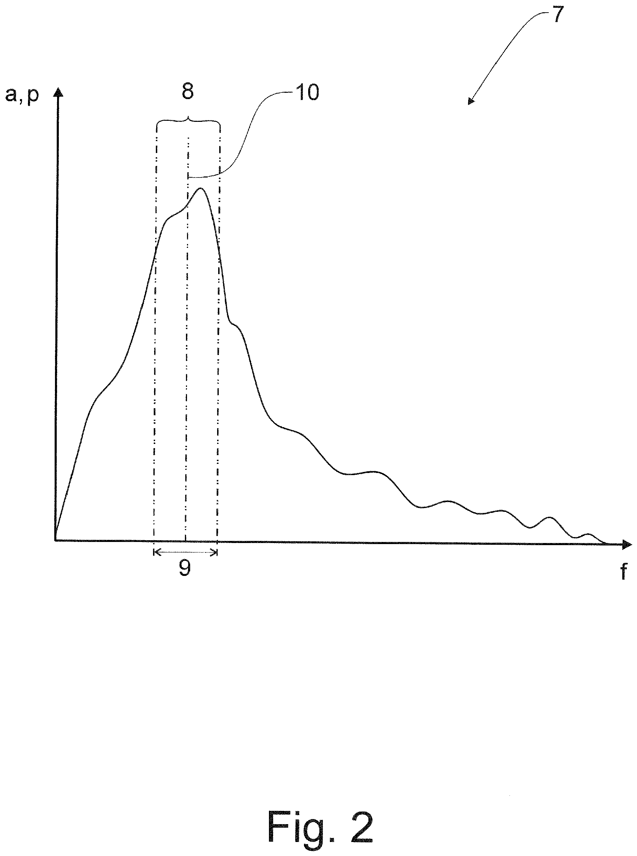

As a further step, preferably subsequent step to the removal step 3, the method comprises a decomposition step which, due to its abstract nature, is not shown in Fig. 1. In the decomposition step, the control system 2 decomposes the ECG data 1 by a frequency decomposition thereby generating frequency data 7 shown in Fig. 2. The frequency data 7 may in particular be a frequency spectrum as shown. The frequency data 7 comprises a frequency dimension f and an amplitude dimension a. The frequency decomposition may be any suitable frequency decomposition. The frequency dimension f may be separated into scales instead of "frequencies" according to the narrow physical meaning. The amplitude dimension a may represent a physical parameter like power density but may also be a purely theoretical dimension.

It is essential that in an analysis step, which due to its abstract nature is also not shown in Fig. 1 as such, the control system 2 identifies in the frequency data 7 a dominant window 8 having a width 9 in the frequency dimension f and meeting an amplitude criterion. The dominant window 8 is a part of the frequency dimension f found by looking for the amplitude criterion. The width 9 of the dominant window 8 may be pre-defined or variable. The dominant window 8 includes at least two, preferably at least 5, more preferably at least 10, frequencies or frequency bins in the frequency dimension f.

It is further essential, that the amplitude criterion is based on a sum of amplitudes inside the dominant window 8. Here and in the following the term "sum" includes calculating an integral along the frequency dimension f even though the amplitudes will usually be defined discreetly. It may also comprise any other mathematical concept leading to a value representing the sum, like calculating a mean value and the like. In the preferred embodiment, the sum of amplitudes is based on a real addition of the amplitudes and/or is the sum of all amplitudes inside the dominant window 8.

It is further essential, that the control system 2 identifies the dominant window 8 by searching on at least a section, in particular a pre-defined section, of the frequency dimension f for a window fulfilling the amplitude criterion. The search may be conducted on the whole frequency dimension f. The control system 2 may use any suitable search method. The search method may be optimized for the amplitude criterion to avoid more complex calculations on parts of the frequency dimension f that are easily excludable. The pre-defined section of the frequency dimension f may, for example, exclude non-physiological frequencies, for example, frequencies higher than 10Hz.

It is further essential that based on the dominant window 8 the control system 2 determines the classification of atrial fibrillation in the ECG data 1.

Here and preferably, the amplitude dimension a is an energy density or power density dimension p. This dimension may be normalized or otherwise processed without changing its information content. In the embodiment shown in Fig. 2 and preferred as such, the frequency data 7 comprises a power spectral density spectrum of the ECG data 1.

The frequency decomposition may additionally or alternatively comprise a Fourier transformation and/or a Welch method. The Fourier transformation and the Welch method are well known to the person skilled in the art. The frequency decomposition may also comprise a model-based decomposition, preferably an autoregressive moving average based decomposition. Such a model-based decomposition can be adapted specifically to the atrial signal data 5. It is evident that presently a broad definition of "frequency" is used, including scales, in particular Wavelet scales, and the like.

The frequency decomposition may comprise a time-frequency decomposition with a time dimension, in particular a short-time Fourier transformation. The time-frequency decomposition may also comprise a wavelet transformation, preferably a wavelet transformation based on a Gaussian wavelet, more preferably a first or second derivative of a Gaussian wavelet. The wavelet transformation may be scaled according to an expected dominant window 8. It may also be applied in different scales subsequently during searching for the dominant window 8.

By calculating multiple frequency decompositions along the time dimension of the ECG data 1 rapidly changing transient components of the atrial activation can be identified and analyzed and/or removed. For the frequency-decomposition, in particular the time-frequency decomposition, a window function, in particular, a Hamming window, may be used. The time-frequency decomposition may be, at least partially, averaged over the time dimension.

With regards to Fig. 2, the amplitude criterion in the preferred embodiment will now be explained. The amplitude criterion here and preferably comprises the primary criterion that a sum of amplitudes in the dominant window 8 is at least equal to a percentage of a sum of amplitudes along a part of the frequency dimension f. This can be better understood by way of example. Here, the dominant window 8 is the smallest window comprising for example 15 percent of the total power of the power spectral density spectrum of the ECG data 1.

Based on this example but speaking generally, the percentage of the sum of amplitudes may be pre-defined. It may be constant, for example always 15 percent but may also depend on the ECG data 1, in particular, the atrial fibrillation data 5 and then, for example, the total power of the power spectral density spectrum. It may be mentioned again that in a continuous spectrum like the one shown in Fig. 2 the sum could be an integral. The percentage of the sum of amplitudes can be a percentage of the total frequency dimension f and it may be pre-defined. As an alternative, the part of the frequency dimension f could comprise only a physiological part or any other part of the frequency dimension f chosen in particular to exclude possible problem frequencies like powerline interference frequencies or harmonics.

The primary criterion may also be that a sum of amplitudes in the dominant window 8 and harmonic frequencies of the dominant window is at least equal to a percentage of a sum of amplitudes along a part of the frequency dimension f.

The amplitude criterion may comprise a boundary condition. The boundary condition can be an absolute condition like the amplitude dropping below a pre-defined value or a value defined relative to the total power or a power peak or the like. Preferably the boundary condition is a differential boundary condition and preferably the boundary of the dominant window 8 is set to a local minimum.

The amplitude criterion may further or alternatively comprise the secondary criterion that the dominant window 8 is the smallest window for fulfilling the primary criterion. With all criteria, the required search precision may be reduced.

After finding the dominant window 8 the control system 2 may conduct further analysis. Preferably the control system 2 identifies a dominant frequency 10 based on and in particular in the dominant window 8 and classifies the atrial fibrillation based on the dominant frequency 10. For example, known methods to use the dominant frequency 10 may be used with this newly identified dominant frequency 10. In the embodiment shown in Fig. 2, the dominant frequency 10 is the centre, defined as the middle between the boundaries, of the dominant window 8. It may alternatively be the boundary of a, in particular pre-defined, percentile of the amplitudes of the dominant window 8 or a frequency associated with an average value, in particular the mean value or the median value, of the amplitudes of the dominant window 8. As can be seen in Fig. 2, in this example the dominant frequency 10 is not the peak frequency, leading to a different result than known algorithms.

In general, the control system 2 may carry out a plausibility check for the dominant window 8 and/or the dominant frequency 10. This plausibility check comprises checking if the dominant window 8 and/or the dominant frequency 10 are inside a physiologically plausible frequency band. If the plausibility check is negative, the control system 2 may resort to a different search method and in particular identify a second-best candidate as the dominant window 8.

In the cases where the width 9 of the dominant window 8 is not pre-defined as a constant, it may be the case that the control system 2 analyzes the width 9 of the dominant window 8 and classifies the atrial fibrillation based on this analysis. Reverting to the example, if the dominant window 8 is the smallest window fulfilling the amplitude criterion, a small dominant window 8 may be indicative for low complexity of atrial fibrillation.

The control system 2 can identify harmonic dominant windows as multiples of the frequency values of the dominant window 8 and here and preferably analyzes the harmonic dominant windows and classifies the atrial fibrillation based on the harmonic dominant windows. For example, a high power density inside the harmonic dominant windows could be used as an indicator for a good dominant window 8 estimation. Not being able to identify harmonic dominant windows could be an indicator for a high signal complexity which could be based on bad signal processing, but also on high atrial fibrillation complexity. These reasons can be separated by further analysis.

Here and preferably the control system 2 analyses skewness and/or kurtosis of the amplitude dimension a inside the dominant window 8, preferably around the dominant frequency 10, and classifies the atrial fibrillation based on the skewness and/or kurtosis.

If the method is applied to ECG data 1 of users U that have not yet been diagnosed in relation to atrial fibrillation, it may be interesting to diagnose atrial fibrillation in a first step. Hence, in such a case, the classification of atrial fibrillation may comprise a presence or absence of the atrial fibrillation. The classification of the atrial fibrillation may further or alternatively comprise a severity indicator of the atrial fibrillation. This severity indicator may comprise a likelihood of success of ablation therapy. The method could also be used for a long time ECG to analyze the frequency of atrial fibrillation episodes and their duration. The classification may be or include a quantification of atrial fibrillation, in particular the severity indicator.

As indicated in Fig. 1, the ECG data 1 may be body surface ECG data. Other preferred embodiments include the ECG data 1 being intracardiac or subcutaneous ECG data 1. If the ECG data 1 is intracardiac data, its measurement may preferably be unipolar or bipolar.

The control system 2 may comprise an electrical input interface 11 connectable to electrodes 12 and adapted to measure the ECG data 1. In Fig. 1 there are shown only three electrodes 12 which are used to measure a single-channel ECG. However, other ECGs and in particular a 12-channel ECG may be used. The ECG data 1 can then be one of those channels or a mixture, particularly a mean value, of multiple channels. Another preferred possibility is that the electrodes 12 are part of a wearable, in particular a smartwatch, and/or a smartphone and/or an implant and/or for the ECG data 1 to be ECG data 1 measured by a wearable and/or smartphone and/or implant.

Here and preferably the control system 2 comprises electrodes 12 connected to the electrical input interface 11 and a patient, here the user U. It is preferred that the surface ECG data 1 is measured via the electrodes 12.

Also shown in Fig. 1 is a pre-processing step 13. It is preferred that in the pre-processing step 13 the control system 2 processes the ECG data 1 to remove noise, preferably by using non-linear filtering, in particular median filtering, and/or to remove powerline interference data 14, in particular at 50Hz or 60Hz and harmonic frequencies of 50Hz or 60Hz, preferably using an adaptive notch filter. As shown, the pre-processing is preferably done prior to the ventricular component removal step 3 and/or the decomposition step.

The ventricular component removal step 3 here and preferably comprises a QRS removal and preferably a T-wave filtering. The QRS removal is preferably based on the subtraction of a template 6 of a QRS complex. This template 6 may be subtracted from the ECG data 1 and the time domain. It has been found that in some instances a QRS removal is advantageous over a QRST removal. The T-wave is not always fully dependent on the QRS complex. Especially when using a template 6 for subtracting it can be advantageous to only subtract the QRS complex and filter the T-wave by a different method or with a different template 6. Here and preferably the T-wave filtering is a non-linear t-wave filtering.

In other instances, the ventricular component removal step 3 may comprise a QRST removal, preferably also based on a template 6. As morphologies of ECG data 1 vary between different persons, it may also be advantageous to implement both methods and compare the results for a single user U.

Shown in the left bottom part of Fig. 1 is the preferred embodiment that the QRS removal or QRST removal comprises a grouping of QRS or QRST complexes contained in the ECG data 1 into at least two different morphology groups, creating a QRS or QRST template 15 for at least two of the morphology groups and removing the QRS or QRST complexes of the respective morphology groups with the respective templates 15. In Fig. 1, there are shown three different templates 15. The necessary number of templates 15 may be detected automatically.

Further to the analysis described above, the control system 2 may analyze the ECG data 1 in the time domain using a time domain analysis after removing the ventricular components 4 and additionally classifies the atrial fibrillation based on the time domain analysis. Preferably the time domain analysis comprises a sample entropy analysis and/or a principle component analysis and/or a wave amplitude analysis and/or a wave correlation analysis. These may be used to generally judge the complexity of the atrial fibrillation in the time domain.

According to another teaching, a control system 2 for performing the proposed method is proposed. Reference is made to all explanations given before. The control system 2 may comprise the electrical input interface 11 and preferably electrodes 12.

### Reference numerals

- 1: ECG data
- 2: control system
- 3: ventricular component removal step
- 4: ventricular components
- 5: atrial signal data
- 6: template
- 7: frequency data
- 8: dominant window
- 9: width
- 10: dominant frequency
- 11: electrical input interface
- 12: electrodes
- 13: pre-processing step
- 14: powerline interference data
- 15: QRS template
- U: user
- f: frequency dimension
- a: amplitude dimension
- p: power density dimension

## Claims

1. Method for classifying atrial fibrillation by analysing ECG data (1) via a control system (2),
wherein in a decomposition step the control system (2) decomposes the ECG data (1) by a frequency decomposition thereby generating frequency data (7), in particular a frequency spectrum, comprising a frequency dimension (f) and an amplitude dimension (a),
**characterized in**
**that** in an analysis step the control system (2) identifies in the frequency data (7) a dominant window (8) having a width (9) in the frequency dimension (f) and meeting an amplitude criterion, that the dominant window (8) includes at least two frequencies or frequency bins in the frequency dimension (f), that the amplitude criterion is based on a sum of amplitudes inside the dominant window (8), that the control system (2) identifies the dominant window (8) by searching on at least a section, in particular a pre-defined section, of the frequency dimension (f) for a window fulfilling the amplitude criterion and
**that** based on the dominant window (8) the control system (2) determines the classification of atrial fibrillation in the ECG data (1).

2. Method according to claim 1, **characterized in that** in a ventricular component removal step (3) the control system (2) processes the ECG data (1) to remove ventricular components (4) from the ECG data (1).

3. Method according to claim 1 or 2, **characterized in that** the amplitude dimension (a) is an energy density or power density dimension (p), preferably, that the frequency data (7) comprises a power spectral density spectrum of the ECG data (1).

4. Method according to one of the preceding claims, **characterized in that** the frequency decomposition comprises a Fourier transformation, and/or, that the frequency decomposition comprises a Welch method, and/or, that the frequency decomposition comprises a model-based decomposition, preferably an autoregressive moving average based decomposition.

5. Method according to one of the preceding claims, **characterized in that** the frequency decomposition comprises a time-frequency decomposition with a time dimension, in particular a short time Fourier transformation and/or a Wavelet transformation, preferably that the time-frequency decomposition is, at least partly, averaged over the time dimension.

6. Method according to one of the preceding claims, **characterized in that** the amplitude criterion comprises the primary criterion that a sum of amplitudes in the dominant window (8) is at least equal to an, in particular pre-defined, percentage of a sum of amplitudes along an, in particular pre-defined, part of the frequency dimension (f), preferably the total frequency dimension (f).

7. Method according to claim 6, **characterized in that** the amplitude criterion comprises a boundary condition, in particular a differential boundary condition, and/or, that the amplitude criterion comprises the secondary criterion that the dominant window (8) is the smallest window fulfilling the primary criterion.

8. Method according to one of the preceding claims, **characterized in that** the control system (2) identifies a dominant frequency (10) based on, in particular in, the dominant window (8) and classifies the atrial fibrillation based on the dominant frequency (10), preferably that the dominant frequency (10) is the centre of the dominant window (8) or the boundary of an, in particular pre-defined, percentile of the amplitudes of the dominant window (8) or a frequency associated with an average value, in particular the mean value or the median value, of the amplitudes of the dominant window (8).

9. Method according to one of the preceding claims, **characterized in that** the control system (2) analyses the width (9) of the dominant window (8) and classifies the atrial fibrillation based on this analysis, and/or, that the control system (2) identifies harmonic dominant windows as multiples of the frequency values of the dominant window (8) and analyses the harmonic dominant windows and classifies the atrial fibrillation based on the harmonic dominant windows.

10. Method according to one of the preceding claims, **characterized in that** the control system (2) analyses skewness and/or kurtosis of the amplitude dimension (a) inside the dominant window (8), preferably around the dominant frequency (10), and classifies the atrial fibrillation based on the skewness and/or kurtosis.

11. Method according to one of the preceding claims, **characterized in that** the classification of atrial fibrillation comprises a presence or absence and/or a severity indicator of the atrial fibrillation, preferably that the severity indicator comprises a likelihood of success of ablation therapy

12. Method according to one of the preceding claims, **characterized in that** the ECG data (1) is body surface ECG data (1) and/or intracardiac ECG data (1) and/or subcutaneous ECG data (1), and/or, that the control system (2) comprises an electrical input interface (11) connectable to electrodes (12) and adapted to measure the ECG data (1).

13. Method according to one of the preceding claims, **characterized in that** in a pre-processing step (13) the control system (2) processes the ECG data (1) to remove noise using non-linear filtering, in particular median filtering and/or removing powerline interference data (14), in particular at 50 Hz or 60 Hz and harmonic frequencies of 50 Hz or 60 Hz, preferably using an adaptive notch filter.

14. Method according to one of the preceding claims, **characterized in that** the ventricular component removal step (3) comprises a QRS removal and preferably a T-wave filtering, in particular a non-linear T-wave filtering, or a QRST removal, preferably that the QRS removal or QRST removal comprises a grouping of QRS or QRST complexes contained in the ECG data (1) into at least two different morphology groups, creating a QRS or QRST template (15) for at least two of the morphology groups and removing the QRS or QRST complexes of the respective morphology groups with the respective templates (15).

15. Method according to one of the preceding claims, **characterized in that** the control system (2) analyses the ECG data (1) in the time domain using a time domain analysis after removing the ventricular components (4) and additionally classifies the atrial fibrillation based on the time domain analysis, preferably that the time domain analysis comprises a sample entropy analysis and/or a principal component analysis and/or a wave amplitude analysis and/or a wave correlation analysis.

16. Control system for performing the method according to one of the preceding claims.

## Patentansprüche

1. Verfahren zum Klassifizieren von Vorhofflimmern durch Analysieren von EKG-Daten (1) mittels eines Steuersystems (2),
wobei in einem Zerlegungsschritt das Steuersystem (2) die EKG-Daten (1) durch eine Frequenzzerlegung zerlegt, wodurch Frequenzdaten (7) erzeugt werden, insbesondere ein Frequenzspektrum, die eine Frequenzdimension (f) und eine Amplitudendimension (a) umfassen,
**dadurch gekennzeichnet,**
**dass** in einem Analyseschritt das Steuersystem (2) in den Frequenzdaten (7) ein dominantes Fenster (8) identifiziert, das eine Breite (9) in der Frequenzdimension (f) aufweist und ein Amplitudenkriterium erfüllt, dass das dominante Fenster (8) mindestens zwei Frequenzen oder Frequenzbins in der Frequenzdimension (f) beinhaltet, dass das Amplitudenkriterium auf einer Summe von Amplituden innerhalb des dominanten Fensters (8) basiert, dass das Steuersystem (2) das dominante Fenster (8) durch Suchen in zumindest einem Abschnitt, insbesondere einem vordefinierten Abschnitt, der Frequenzdimension (f) nach einem Fenster, das das Amplitudenkriterium erfüllt, identifiziert, und dass das Steuersystem (2) basierend auf dem dominanten Fenster (8) die Klassifizierung von Vorhofflimmern in den EKG-Daten (1) bestimmt.

2. Verfahren nach Anspruch 1, **dadurch gekennzeichnet, dass** in einem Schritt (3) zum Entfernen der ventrikulären Komponente das Steuersystem (2) die EKG-Daten (1) verarbeitet, um ventrikuläre Komponenten (4) aus den EKG-Daten (1) zu entfernen.

3. Verfahren nach Anspruch 1 oder 2, **dadurch gekennzeichnet, dass** die Amplitudendimension (a) eine Energiedichte- oder Leistungsdichtedimension (p) ist, vorzugsweise dass die Frequenzdaten (7) ein spektrales Leistungsdichtespektrum der EKG-Daten (1) umfassen.

4. Verfahren nach einem der vorstehenden Ansprüche, **dadurch gekennzeichnet, dass** die Frequenzzerlegung eine Fourier-Transformation umfasst und/oder dass die Frequenzzerlegung ein Welch-Verfahren umfasst und/oder dass die Frequenzzerlegung eine modellbasierte Zerlegung umfasst, vorzugsweise eine Zerlegung basierend auf autoregressivem gleitendem Durchschnitt.

5. Verfahren nach einem der vorstehenden Ansprüche, **dadurch gekennzeichnet, dass** die Frequenzzerlegung eine Zeit-Frequenz-Zerlegung mit einer Zeitdimension umfasst, insbesondere eine Kurzzeit-Fourier-Transformation und/oder eine Wavelet-Transformation, vorzugsweise dass die Zeit-Frequenz-Zerlegung zumindest teilweise über die Zeitdimension gemittelt wird.

6. Verfahren nach einem der vorstehenden Ansprüche, **dadurch gekennzeichnet, dass** das Amplitudenkriterium das Primärkriterium umfasst, dass eine Summe von Amplituden im dominanten Fenster (8) zumindest gleich einem, insbesondere vordefinierten, Prozentsatz einer Summe von Amplituden entlang eines, insbesondere vordefinierten, Teils der Frequenzdimension (f) ist, vorzugsweise der Gesamtfrequenzdimension (f).

7. Verfahren nach Anspruch 6, **dadurch gekennzeichnet, dass** das Amplitudenkriterium eine Grenzbedingung umfasst, insbesondere eine differenzielle Grenzbedingung, und/oder dass das Amplitudenkriterium das Sekundärkriterium umfasst, dass das dominante Fenster (8) das kleinste Fenster ist, das das Primärkriterium erfüllt.

8. Verfahren nach einem der vorstehenden Ansprüche, **dadurch gekennzeichnet, dass** das Steuersystem (2) eine dominante Frequenz (10) basierend insbesondere auf dem dominanten Fenster (8) identifiziert und das Vorhofflimmern basierend auf der dominanten Frequenz (10) klassifiziert, vorzugsweise dass die dominante Frequenz (10) die Mitte des dominanten Fensters (8) oder die Grenze eines, insbesondere vordefinierten, Perzentils der Amplituden des dominanten Fensters (8) oder eine Frequenz, die mit einem Durchschnittswert assoziiert ist, insbesondere der Mittelwert oder der Medianwert der Amplituden des dominanten Fensters (8) ist.

9. Verfahren nach einem der vorstehenden Ansprüche, **dadurch gekennzeichnet, dass** das Steuersystem (2) die Breite (9) des dominanten Fensters (8) analysiert und das Vorhofflimmern basierend auf dieser Analyse klassifiziert, und/oder dass das Steuersystem (2) harmonische dominante Fenster als Vielfache der Frequenzwerte des dominanten Fensters (8) identifiziert und die harmonischen dominanten Fenster analysiert und das Vorhofflimmern basierend auf den harmonischen dominanten Fenstern klassifiziert.

10. Verfahren nach einem der vorstehenden Ansprüche, **dadurch gekennzeichnet, dass** das Steuersystem (2) Schiefe und/oder Kurtosis der Amplitudendimension (a) innerhalb des dominanten Fensters (8) analysiert, vorzugsweise etwa bei der dominanten Frequenz (10), und das Vorhofflimmern basierend auf der Schiefe und/oder Kurtosis klassifiziert.

11. Verfahren nach einem der vorstehenden Ansprüche, **dadurch gekennzeichnet, dass** die Klassifizierung von Vorhofflimmern ein Vorhandensein oder Nichtvorhandensein und/oder einen Schwereindikator des Vorhofflimmerns umfasst, vorzugsweise dass der Schwereindikator eine Erfolgswahrscheinlichkeit von Ablationstherapie umfasst.

12. Verfahren nach einem der vorstehenden Ansprüche, **dadurch gekennzeichnet, dass** die EKG-Daten (1) Körperoberflächen-EKG-Daten (1) und/oder intrakardiale EKG-Daten (1) und/oder subkutane EKG-Daten sind (1), und/oder dass das Steuersystem (2) eine elektrische Eingangsschnittstelle (11) umfasst, die mit Elektroden (12) verbindbar ist und zum Messen der EKG-Daten (1) eingerichtet ist.

13. Verfahren nach einem der vorstehenden Ansprüche, **dadurch gekennzeichnet, dass** in einem Vorverarbeitungsschritt (13) das Steuersystem (2) die EKG-Daten (1) verarbeitet, um Rauschen unter Verwendung einer nichtlinearen Filterung zu entfernen, insbesondere Medianfilterung, und/oder Stromleitungsstörungsdaten (14), insbesondere bei 50 Hz oder 60 Hz, und harmonische Frequenzen von 50 Hz oder 60 Hz vorzugsweise unter Verwendung eines adaptiven Kerbfilters entfernt.

14. Verfahren nach einem der vorstehenden Ansprüche, **dadurch gekennzeichnet, dass** der Schritt (3) zum Entfernen der ventrikulären Komponente eine QRS-Entfernung und vorzugsweise eine T-Wellen-Filterung, insbesondere eine nichtlineare T-Wellen-Filterung, oder eine QRST-Entfernung umfasst, vorzugsweise dass die QRS-Entfernung oder die QRST-Entfernung eine Gruppierung von QRS- oder QRST-Komplexen, die in den EKG-Daten (1) enthalten sind, in mindestens zwei unterschiedliche Morphologiegruppen, wodurch eine QRS- oder QRST-Vorlage (15) für mindestens zwei der Morphologiegruppen erzeugt wird, und Entfernen der QRS- oder QRST-Komplexe der jeweiligen Morphologiegruppen mit den jeweiligen Vorlagen (15) umfasst.

15. Verfahren nach einem der vorstehenden Ansprüche, **dadurch gekennzeichnet, dass** das Steuersystem (2) die EKG-Daten (1) in der Zeitdomäne unter Verwendung einer Zeitdomänenanalyse nach dem Entfernen der ventrikulären Komponenten (4) analysiert und zusätzlich das Vorhofflimmern basierend auf der Zeitdomänenanalyse klassifiziert, vorzugsweise dass die Zeitdomänenanalyse eine Probenentropieanalyse und/oder eine Hauptkomponentenanalyse und/oder eine Wellenamplituden-analyse und/oder eine Wellenkorrelationsanalyse umfasst.

16. Steuersystem zum Durchführen des Verfahrens nach einem der vorstehenden Ansprüche.

## Revendications

1. Procédé de classification de fibrillation auriculaire par analyse de données d'ECG (1) par l'intermédiaire d'un système de commande (2),
dans lequel, dans une étape de décomposition, le système de commande (2) décompose les données d'ECG (1) par une décomposition en fréquence, générant ainsi des données de fréquence (7), en particulier un spectre de fréquences, comprenant une dimension de fréquence (f) et une dimension d'amplitude (a),
**caractérisé en ce que**
dans une étape d'analyse, le système de commande (2) identifie dans les données de fréquence (7) une fenêtre dominante (8) ayant une largeur (9) dans la dimension de fréquence (f) et satisfaisant un critère d'amplitude, **en ce que** la fenêtre dominante (8) comprend au moins deux fréquences ou compartiments de fréquences dans la dimension de fréquence (f), **en ce que** le critère d'amplitude est basé sur une somme d'amplitudes à l'intérieur de la fenêtre dominante (8), **en ce que** le système de commande (2) identifie la fenêtre dominante (8) par recherche sur au moins une section, en particulier une section prédéfinie, de la dimension de fréquence (f) d'une fenêtre satisfaisant le critère d'amplitude et **en ce que**, en fonction de la fenêtre dominante (8), le système de commande (2) détermine la classification de fibrillation auriculaire dans les données d'ECG (1).

2. Procédé selon la revendication 1, **caractérisé en ce que** dans une étape de suppression de composantes ventriculaires (3) le système de commande (2) traite les données d'ECG (1) pour éliminer les composantes ventriculaires (4) des données d'ECG (1).

3. Procédé selon la revendication 1 ou 2, **caractérisé en ce que** la dimension d'amplitude (a) est une densité d'énergie ou une dimension de densité de puissance (p), de préférence, **en ce que** les données de fréquence (7) comprennent un spectre de densité spectrale de puissance des données d'ECG (1).

4. Procédé selon l'une des revendications précédentes, **caractérisé en ce que** la décomposition en fréquence comprend une transformation de Fourier, et/ou, **en ce que** la décomposition en fréquence comprend un procédé de Welch, et/ou, **en ce que** la décomposition en fréquence comprend une décomposition basée sur un modèle, de préférence une décomposition autorégressive basée sur une moyenne mobile.

5. Procédé selon l'une des revendications précédentes, **caractérisé en ce que** la décomposition en fréquence comprend une décomposition temps-fréquence avec une dimension temporelle, en particulier une transformation de Fourier à court terme et/ou une transformation en ondelettes, de préférence **en ce que** la décomposition temps-fréquence est, au moins en partie, moyennée sur la dimension temporelle.

6. Procédé selon l'une des revendications précédentes, **caractérisé en ce que** le critère d'amplitude comprend le critère primaire selon lequel une somme d'amplitudes dans la fenêtre dominante (8) est au moins égale à un pourcentage, en particulier prédéfini, d'une somme d'amplitudes le long d'une partie, en particulier prédéfinie, de la dimension de fréquence (f), de préférence la dimension de fréquence totale (f).

7. Procédé selon la revendication 6, **caractérisé en ce que** le critère d'amplitude comprend une condition de limite, en particulier une condition de limite différentielle, et/ou, **en ce que** le critère d'amplitude comprend le critère secondaire selon lequel la fenêtre dominante (8) est la plus petite fenêtre satisfaisant le critère primaire.

8. Procédé selon l'une des revendications précédentes, **caractérisé en ce que** le système de commande (2) identifie une fréquence dominante (10) en fonction, en particulier, de la fenêtre dominante (8) et classe la fibrillation auriculaire en fonction de la fréquence dominante (10), de préférence **en ce que** la fréquence dominante (10) est le centre de la fenêtre dominante (8) ou la limite d'un percentile, en particulier prédéfini, des amplitudes de la fenêtre dominante (8) ou une fréquence associée à une valeur moyenne, en particulier la valeur moyenne ou la valeur médiane, des amplitudes de la fenêtre dominante (8).

9. Procédé selon l'une des revendications précédentes, **caractérisé en ce que** le système de commande (2) analyse la largeur (9) de la fenêtre dominante (8) et classe la fibrillation auriculaire en fonction de cette analyse, et/ou, **en ce que** le système de commande (2) identifie des fenêtres dominantes harmoniques comme des multiples des valeurs de fréquence de la fenêtre dominante (8) et analyse les fenêtres dominantes harmoniques et classe la fibrillation auriculaire en fonction des fenêtres dominantes harmoniques.

10. Procédé selon l'une des revendications précédentes, **caractérisé en ce que** le système de commande (2) analyse l'asymétrie et/ou le kurtosis de la dimension d'amplitude (a) à l'intérieur de la fenêtre dominante (8), de préférence autour de la fréquence dominante (10), et classe la fibrillation auriculaire en fonction de l'asymétrie et/ou du kurtosis.

11. Procédé selon l'une des revendications précédentes, **caractérisé en ce que** la classification de fibrillation auriculaire comprend un indicateur de présence ou d'absence et/ou de gravité de la fibrillation auriculaire, de préférence **en ce que** l'indicateur de gravité comporte une probabilité de succès d'une thérapie par ablation.

12. Procédé selon l'une des revendications précédentes, **caractérisé en ce que** les données d'ECG (1) sont des données d'ECG de surface corporelle (1) et/ou des données d'ECG intracardiaques (1) et/ou des données d'ECG sous-cutanées (1), et/ou, **en ce que** le système de commande (2) comporte une interface d'entrée électrique (11) pouvant être connectée à des électrodes (12) et adaptée pour mesurer les données d'ECG (1).

13. Procédé selon l'une des revendications précédentes, **caractérisé en ce que**, dans une étape de prétraitement (13), le système de commande (2) traite les données d'ECG (1) pour éliminer le bruit par filtrage non linéaire, en particulier un filtrage médian et/ou par suppression de données d'interférence de courants porteurs (14), en particulier à 50 Hz ou 60 Hz et aux fréquences harmoniques de 50 Hz ou 60 Hz, de préférence à l'aide d'un filtre à encoche adaptatif.

14. Procédé selon l'une des revendications précédentes, **caractérisé en ce que** l'étape de suppression de composantes ventriculaires (3) comprend une suppression de QRS et de préférence un filtrage d'onde T, en particulier un filtrage d'onde T non linéaire, ou une suppression de QRST, de préférence **en ce que** l'élimination de QRS ou QRST comprend un regroupement de complexes QRS ou QRST contenus dans les données d'ECG (1) en au moins deux groupes morphologiques différents, créant un modèle QRS ou QRST (15) pour au moins deux des groupes de morphologie et éliminant les complexes QRS ou QRST des groupes de morphologie respectifs avec les modèles respectifs (15).

15. Procédé selon l'une des revendications précédentes, **caractérisé en ce que** le système de commande (2) analyse les données d'ECG (1) dans le domaine temporel à l'aide d'une analyse dans le domaine temporel après la suppression des composantes ventriculaires (4) et classe de plus la fibrillation auriculaire en fonction de l'analyse dans le domaine temporel, de préférence **en ce que** l'analyse dans le domaine temporel comprend une analyse entropique d'échantillon et/ou une analyse de composantes principales et/ou une analyse d'amplitude des ondes et/ou une analyse de corrélation des ondes.

16. Système de commande pour réaliser le procédé selon l'une des revendications précédentes.
